# EUROPEAN PATENT APPLICATION

(11) **EP 0 535 241 A1**
(43) Date of publication of application: **07.04.1993**
(21) Application number: 92907603.2
(22) Date of filing: 31.03.1992
(51) Int. Cl.: C12N 9/48

(54) **NOVEL LEUCINE AMINOPEPTIDASE**

(30) Priority: 08.04.1991 JP 160186/91
(71) Applicant: SUNTORY LIMITED, Kita-ku, Osaka-shi, Osaka 530 (JP)
(72) Inventor: TSUJIMOTO, Masafumi, Mishima-gun, Osaka 618 (JP); ADACHI, Hideki, Takatsuki-shi, Osaka 569 (JP); NAKAZATO, Hiroshi, Ibaraki-shi, Osaka 567 (JP)
(74) Representative: Stoner, Gerard Patrick
(86) International application number: JP9200398
(87) International publication number: WO9217575

(57) **Abstract**

A novel leucine aminopeptidase having the following properties: (a) action and substrate specificity: (1) having an activity of decomposing a synthetic substrate, L-leucine P-nitroanilide, characterized by having a methionine resistance, (2) having a cystine aminopeptidase activity, and (3) having an activity of decomposing at least oxytocin, vasopressin and angiotensin III, among naturally occurring hormones; (b) optimum pH and stable pH range: having the optimum pH around a neutral region (at a pH of about 7.4); (c) condition of deactivation by pH, temperature, etc.: being deactivated at 70 °C in 30 minutes; (d) inhibition, activation and stabilization: being deactivated by a divalent metallic ion such as Zn²⁺, Co²⁺ or Ni²⁺; (e) molecular weight: having a molecular weight of about 490 kDa (according to gel filtration), while that of the subunit being about 210 kDa (according to SDS-PAGE) (no difference in mobility observed between reducing and nonreducing conditions); (f) sequence number: having the following amino acid sequences represented by 1,2,3 and 4: Gly Trp Gly Leu Gly Lys; Leu Leu Gln Asn Gln Ile Gln Gln Gln Thr Arg Thr Asp Glu Gly Thr Pro; Thr Asp Lys Gly Trp Ser Phe Leu Leu Gly Lys; Phe Phe Leu Asn Met Lys Pro Glu Ile Gln Pro Gln Asp; (g) purification: being purified from the human retroplacental blood after eutocia.

## Description

### TECHNICAL FIELD

The present invention relates to leucine aminopeptidase of human placenta origin (hereinafter, abbreviated as PLAP).

### BACKGROUND ART

Although it is known that various aminopeptidases (AP) are present in the placenta and some of them are released into the blood of the maternal body, the physiological significance thereof is not understood completely. It is known that various peptide hormones are present in the fetus-placenta system, and it is considered that they play important roles in rapid growth and maturation in the fetus phase. Therefore it can be expected that abnormal production of these hormones is disadvantageous for the growth of the fetus and the stability of the maternal body, and there is the possibility that a group of APs occurring in the placenta act on various peptide hormones to control actions thereof.

The AP, which is known as placenta leucine aminopeptidase (PLAP), increases as the pregnancy progresses, and rapidly disappears after delivery, and an abnormal serum level thereof occurs in abnormal pregnancy such as a threatened abortion, and this enzyme probably acts to degrade oxytocin, which acts to contract the uterus, as reported by Mizutani et al. (Rinsho Fujin-ka San-ka in Japanese 43, p1045-1054, 1989). Therefore, it is expected that the enzyme plays an important role in maintaining the pregnancy and inducing delivery. Moreover, although it is shown that activity of the enzyme, different from that of leucine aminopeptidase present in serum, is not effected by L-methionine (Oya et al., Experimentia, 30, 985-986, 1976), it has not yet been purified and its structure has not been clarified. Accordingly, the present invention includes a technique for purifying the enzyme having the above-mentioned properties to a homologous state, and provides the possibility of use thereof as a medicament for inhibiting abortion.

### DISCLOSURE OF THE INVENTION

The present invention provides an isolated leucine aminopeptidase of human placenta origin, having the following properties:
A) Action and substrate specificity:
   1) acts to degradate a synthetic substrate L-leucyl-P-nitroanilide, and this activity being not inhibited by L-methionine;
   2) acts to degradate at least oxytocin, vasopressin and angiotensin III among naturally occurring hormones;
B) Optimum pH and stable pH range: having an optimum pH in a neutral range (pH about 7.4);
C) Condition for inactivation by pH and temperature: in activated at 70°C (30 minutes);
D) Inhibition, activation and stabilization: inactivated by divalent metal ions such as Zn²⁺, Co²⁺, Ni²⁺, or the like;
E) Molecular weight: about 49O kDa (by gel filtration method); subunit, about 210 kDa (by SDS-PAGE method) (there is no difference in mobility between reducing condition and non-reducing condition); and
F) containing the amino acid sequences shown in SEQ ID NOs: 1, 2, 3 and 4.

### BRIEF EXPLANATION OF DRAWING

Fig. 1 shows a fraction of PLAP by a DEAE-Sepharose column, wherein ( ⃝) shows an elution pattern of proteins, and (●) shows PLAP activity.

Fig. 2 shows a fraction of PLAP by a phenyl-Sepharose column, wherein ( ⃝) shows an elution pattern of proteins, and (●) shows PLAP activity.

Fig. 3 shows a fraction of PLAP by a Sepharose CL6B column, wherein ( ⃝) shows an elution pattern of PLAP, and (●) shows PLAP activity. The arrows show elution positions of the following standard proteins:
1: thyroglobulin (669 kDa), ferritin (440 kDa), 3: catalase (232 kDa).

Fig. 4 represents the result of an electrophoresis showing an analysis of a purified PLAP by SDS-PAGE (12.5% gel), wherein lane 1 shows the result for placenta blood (starting material), and lane 2 shows the result for purified PLAP.

Fig. 5A represents the result of electrophoresis showing a behavior of 210 kDa band by SDS-PAGE. Fig. 5B shows a comparison of elution patterns of PLAP and CAP activities in gel filtration, wherein (●) shows PLAP activity, and (□) shows CAP activity.

Fig. 6 is a figure showing the pH-dependency of PLAP, wherein (●) shows the result of a measurement using 20 mM phosphate buffer, and ( ⃝) shows the result of measurement using a 20 mM Tris/HCl buffer.

Fig. 7 is a figure showing heat stability of PLAP and residual activity after treatment for 30 minutes at each temperature.

Fig. 8 is a figure showing residual activities in the presence at various metal ions (5 mM).

Fig. 9 is a figure showing activity of purified PLAP degrading various peptide hormones.

Fig. 10 shows amino acid sequences of various peptide hormones, wherein the arrows show sites cleaved by PLAP.

### Detailed Description

### (1) Materials

Human retroplacental sera was obtained from individuals after normal delivery, pooled and frozen at -80°C to use a starting material for the purification of PLAL. L-leucyl-P-nitroanilide was purchased from Wako Pure Chemical, and an assay reagent for cysteine aminopeptidase (CAP Color Test) was purchased from Sankyo.

### (2) Enzyme assay

An assay of PLAP was carried out according to a method of Kurauchi et al. (Kurauchi, O. et al. Enzyme, 35, p197-205, 1986) using L-leucyl-P-nitroanilide as a substrate. Namely, a substrate adjusted to 1.6 mM of a final concentration was added to a 20 mM Tris-HCl buffer (pH 7.2) (1.0 ml) in the presence of a 20 mM L-methionine, and the mixture was incubated at 37°C for 10 minutes. After finishing the reaction, 3.0 ml of a 1M acetate buffer (pH 4.2) was added to terminate the reaction, and the amount of released P-nitroanilide was measured by absorption of 405 nm. One unit of the enzyme is defined as an activity of the enzyme that releases 1 µ mole of the P-nitroanilide for one minute.

The activity of cysteine aminopeptidase was measured by a CAP Color Test using S-benzyl-L-cysteine-P-dimethylaminoanilide as a substrate. The procedure followed operation method attached to the reagent, and enzyme activity was determined by measuring the amount of released P-nitroanilide on the basis of absorbance at 660 nm.

The degradation of various peptide hormones was tested according to a Mizutani et al. (Mizutani, S. et al. Exp. Clin. Endocrinal. 86, p310-316, 1985). Namely, a hormone was dissolved in a 50 mM phosphate buffer (pH 7.4) containing 0.15M NaCl, and the solution (20 µl), an enzyme solution (10 µl) and a phosphate buffer (40 µl) were mixed, and the mixture was then incubated at 37°C. After incubation for an appropriate amount of time, 70 µl of ethanol was added to terminate the reaction, 10 µl of the reaction mixture was separated by HPLC (Shimazu LSC, φ4 × 15 mm), and the amino acids released during the incubation was identified and quantitatively measured.

### (3) Purification of PLAP

PLAP was purified by fractionating 900 ml of retroplacental sera by ammonium sulfate fractionation, obtaining a fraction precipitated at 30 to 60% saturation, and subjecting to a series of column chromatography using Matrex Blue A (Amicon), DEAE-Sepharose (Pharmacia), phenyl 1-Sepharose (Pharmacia), Zn²⁺- chelating Sepharose (Pharmacia) and Sepharose CL6B (Pharmacia). Quantitative analysis of protein was carried out according to Bradford's method (Anal. Biochem, vol72, p248, 1976).

### (4) Carboxy methylation and fragmentation of PLAP

For determination of an amino acid sequence, purified PLAP (200 µg) was carboxy methylated by the McWherler et al. method (McWherler C.A., et al., Anal. Chem. 141, 523-527, 1984), and fragmented by treating, at a ratio of 50:1 at 37°C for 16 hours, with trypsin which had been treated with L-1-tosylamido-2-phenylethyl chloromethyl ketone (TPCK).

The resulting peptide fragments were separated and recovered by reversed phase HPLC (acetonitrile gradient in the presence of 0.1% trifluoroacetic acid) using a Vydac C4 column (Vydac).

### (5) Determination of amino acid sequence

Amino acid sequences of the recovered peptide fragment were determined by an Applied Biosystem's gas-phase sequences.

### EXAMPLES

The present inventions is described in detail by Examples hereinafter.

### Example 1. Purification of PLAP

Ammonium sulfate was added to retroplacental sera to 30% saturation, and after allowing to stand at 4°C for 3 hours, the mixture was centrifuged to obtain a supernatant, to which ammonium sulfate was then added to 60% saturation, and the mixture was allowed to stand for a day. The resulting precipitate was recovered by centrifugation, and repeatedly dialyzed in a 20 mM Tris/HCl buffer (pH 7.4). The PLAP fraction thus obtained was fractionated by applying to a Matrex Blue A column equilibrated with a buffer having the same composition. PLAP activity was recovered in non-binding fractions.

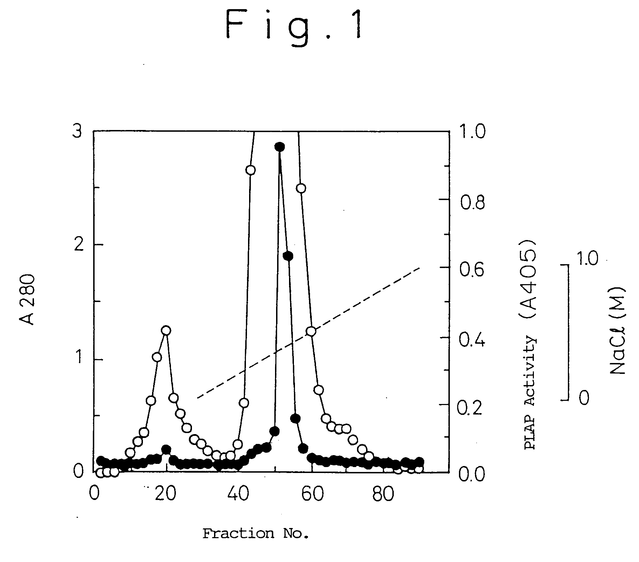

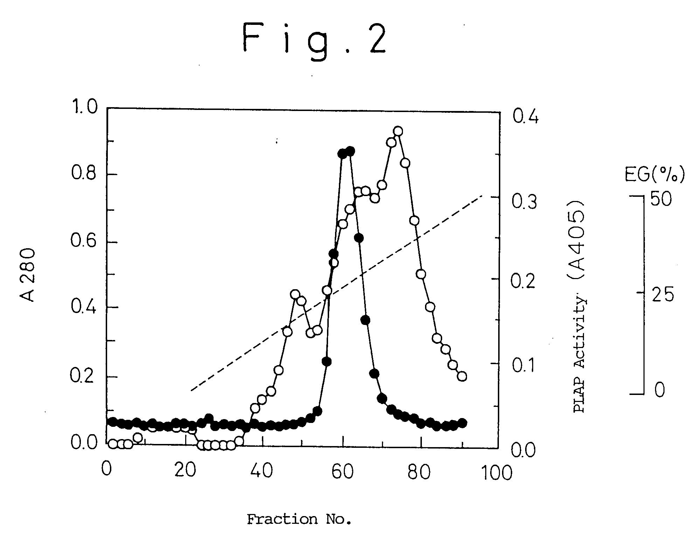

The fractions were applied to a DEAE-Sepharose column equilibrated with 20 mM Tris/HCl buffer (pH 7.4), and after thoroughly washing the column, PLAP was eluted by a NaCl gradient (see, Fig. 1). PLAP was eluted as a single peak around NaCl 0.3M fractions. Ammonium sulfate was added to the PLAP fraction to 30% saturation, and the mixture was applied to a phenyl-Sepharose column equilibrated with a 20 mM Tris/HCl (pH 7.4) buffer containing 30% saturation of ammonium sulfate. PLAP was eluted by forming a co-concentration gradient of ammonium sulfate (30% - 0%) and ethylene glycol (0% - 50%) (see, Fig. 2). PLAP was eluted as a single peak at around ammonium sulfate 50% saturation.

The PLAP fraction thus obtained was thoroughly dialyzed in a 20 mM Tris/HCl buffer (pH 7.4), and applied to a chelating-sepharose column. The chelating sepharose column had been previously loaded with ZnCl₂ and equilibrated with a 20 mM Tris/HCl buffer (pH 7.4). PLAP was recovered in non-bonding fraction.

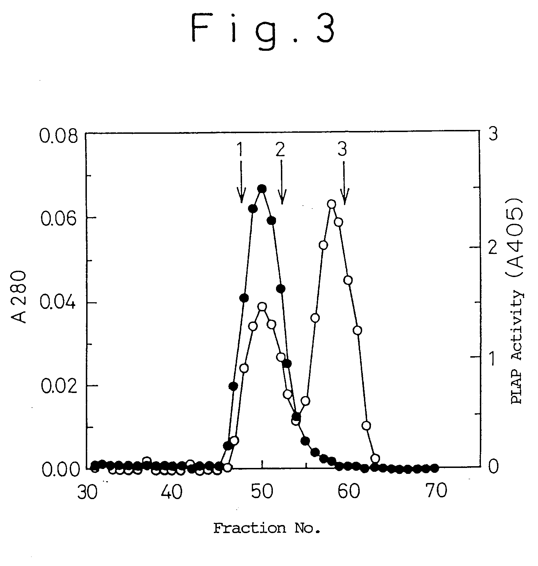

In this case, since most of the proteins was bound to the column, this step was most successful for purification of the present enzyme. The PLAC fraction thus obtained was dialyzed in a 20 mM Tris/HCl buffer, and after concentrating with YM-10, fractionated by a Sepharose CL-6B column equilibrated with a buffer having the same composition (see, Fig. 3). PLAP was eluted as a single band at around 490 kDa.

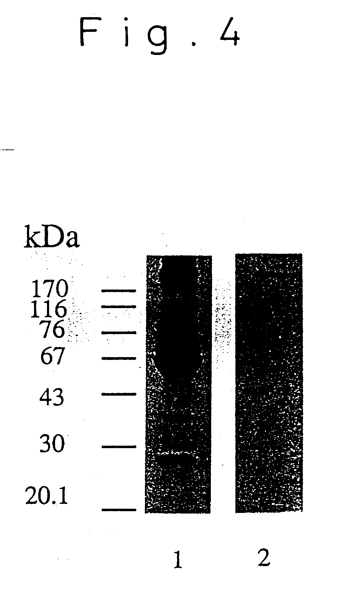

By the above-mentioned steps, a protein fraction showing a single band at around 210 kDa was obtained. The difference in molecular weight obtained by PAGE analysis and that obtained by gel filtration suggests that the present enzyme is a dimer. The result of SDS-PAGE of a starting material and a finally purified preparation is shown in Fig. 4. As shown in Table 1, specific activity of the enzyme increased about 3000 times by the present purification process, and recovery of activity was 15%.

**Table 1**

| | Total protein (mg) | Specific activity (mU/mg) | Total activity (U) | Purification ratio | Recovery (%) |
|---|---|---|---|---|---|
| Serum | 53100 | 4.2 | 225 | 1 | - |
| Ammonium sulfate fraction | 29325 | 5.9 | 172 | 1.4 | 76.4 |
| Matrex Blue A | 12300 | 14.2 | 174 | 3.4 | 77.3 |
| DEAE-Sepharose | 5610 | 17.0 | 95.2 | 4.0 | 42.4 |
| Phenyl-Sepharose | 2224 | 39.1 | 87.0 | 9.3 | 38.7 |
| Chelating-Sepharose | 66 | 915 | 60.4 | 218 | 27.2 |
| Sepharose CL6B | 2.6 | 12960 | 33.7 | 3086 | 15.0 |

### Example 2. Properties of PLAP

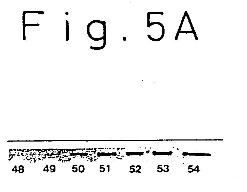

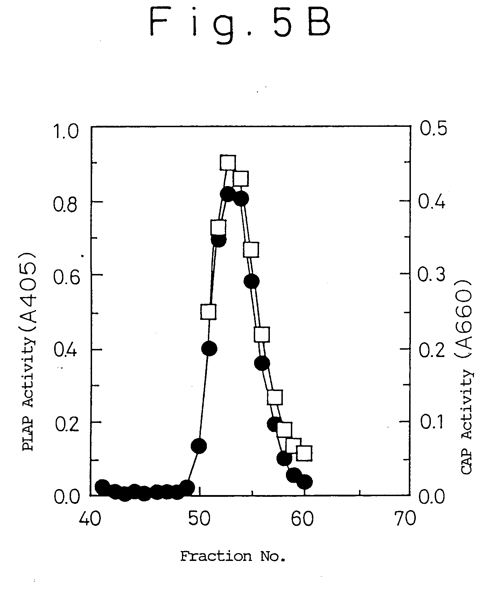

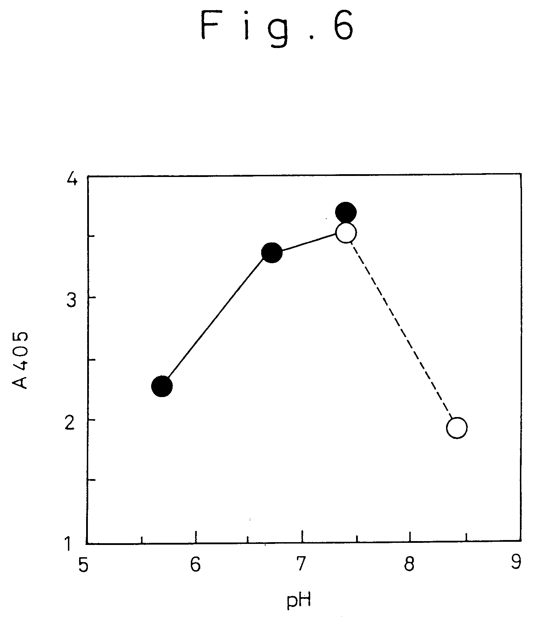

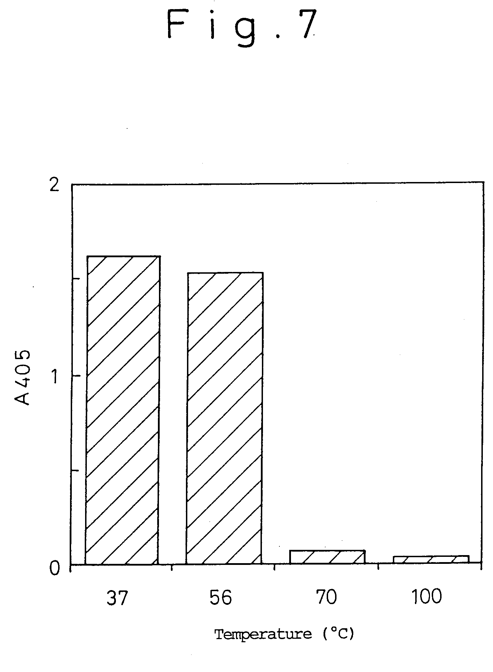

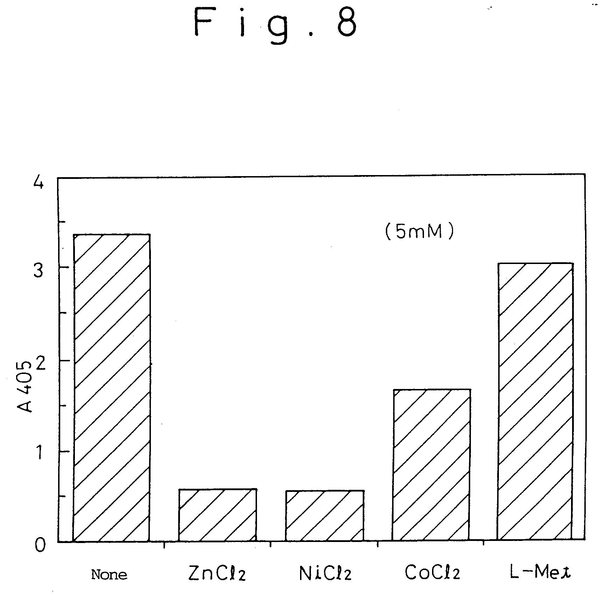

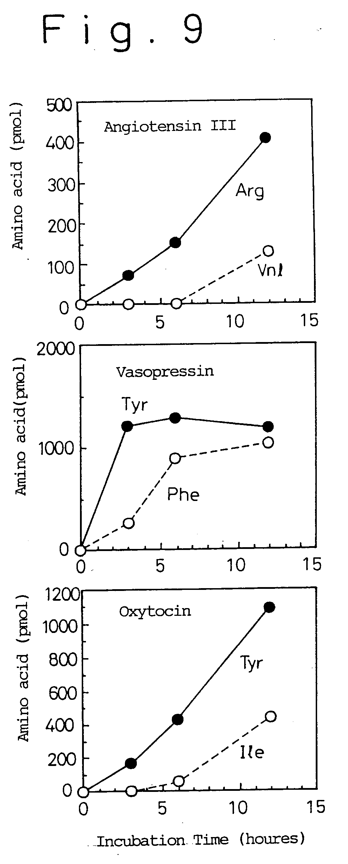

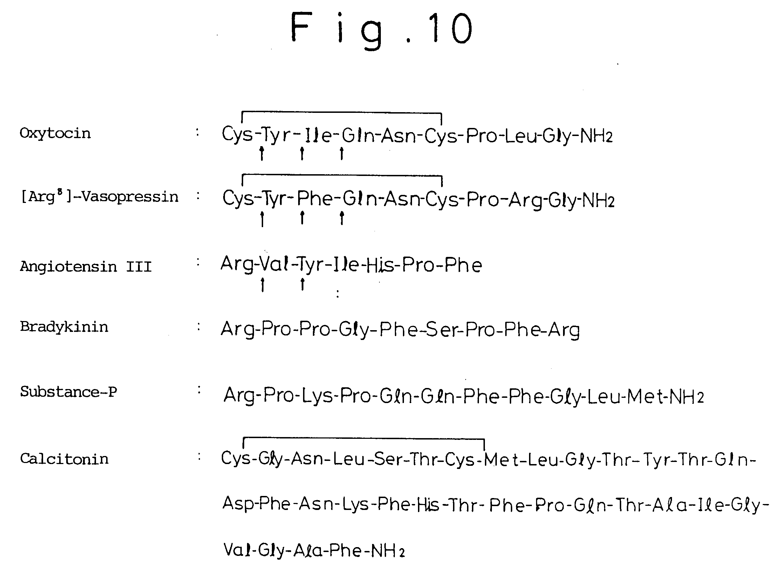

The present enzyme has the following properties:
1) Action and substrate specificity
   The present enzyme is characterized by being resistant to methionine while leucine aminopeptidase present in normal serum (namely not specific to the pregnancy) is sensitive to methionine, and acts to degrade a synthetic substrate L-leucyl-P-nitroanilide. Moreover, when the presence or absence of cysteine aminopeptidase (CAP) activity was tested, the CAP activity showed the same elution pattern as that of PLAP activity (see, Fig. 5B).
   Accordingly, the present enzyme has CAP activity (as a result of analysis by SDS-PAGE, the protein band having a molecular weight of about 210 kDa showed a very high correlation with the behavior of the activity (see, Fig. A)). Moreover, when the activity of purified PLAP on various peptide hormones was tested, the PLAP exhibited the ability to decompose oxytocin, vasopressin and angiotensin III among naturally occurring hormones tested (see, Figs. 9 and 10).
2) Optimum pH and stable pH range:
   The enzyme has optimum pH in a neutral range (Max: 7.4) (Fig. 6).
3) Condition for inactivation by pH, temperature etc.
   The enzyme is relatively stable up to about 56°C, and inactivated at 70°C (30 minutes) (see, Fig. 7).
4) Inhibition, Activation and Stabilization:
   The enzyme is inactivated by divalent metal ions such as Zn²⁺, Co²⁺, Ni²⁺ or the like having a concentration of up to 5 mM (see, Fig. 8).
5) Molecular weight:
   About 490 kDa (gel filtration) (see, Fig. 3); subunit about 210 kDa (SDS-PAGE) (see, Fig. 4). There is no difference in mobilities under reducing conditions and non-reducing conditions.
6) Km value and Vmax:
   A Km value and Vmax for degradation of L-Leucyl-P-nitroanilide by purified PLAP were calculated according to Lineweaver-Burk plot, and were 4.17 mM and 108 µmol/min/g, respectively.
7) The present enzyme contains the amino acid sequences shown by SEQ ID NOs 1 to 4 (see, the following Example 3).

### Example 3. Structure of PLAP

To analyze the structure of the present enzyme, the present enzyme was digested with trypsin and determination of the structure of each peptide fragment was attempted. After tryptic digestion, each peptide fragment was recovered by reversed phase HPLC, and the structure of appropriate fractions was determined. As a result, each peptide fragment has an amino acid sequence shown by one of the SEQ ID Nos: 1 to 4. As a result of a search by an NBRF protein data base, no protein having a sequence matching this amino acid sequence was recognized. Therefore, it is considered that the purified enzyme is an enzyme of a novel protein.

PLAP was purified from human retroplacental sera, and properties and partial structure thereof were determined. The finally purified preparation was found to have leucine peptidase activity and cysteine aminopeptidase activity, as well as activities to degrade oxytocin having uterine musle contracting activity, and vasopressin and angiotensin III having hypertensive action. PLAP increases as the pregnancy progresses, and rapidly disappears after delivery. Moreover, it is known that a level of the enzyme is lower than the normal level in the case of gestational toxicosis and abortion, and recover to a normal range in the group after successful convalescence while it lowers as the symptoms worsen in the group in the case of unsuccessful convalescence. Accordingly, PLAP is considered to play an important role in maintaining a pregnancy.

### [Industrial Applicability]

It is considered that the present enzyme contributes to maintaining an environment for the growth of a fetus by preventing contraction of the uterus, especially due to oxytocin degrading activity. Degradation of vasopressin and angiotensin III suggests that the present enzyme has an important control function in hypertension clinically probremable during the pregnancy. Accordingly, since the present enzyme is possibly essential for maintaining normal pregnancy, it promises to be useful as a drug for preventing threatened abortion.

SEQUENCE LISTINGS
SEQ ID NO: 1
Sequence type: Amino acid
Sequence length: 6
Topology: Linear
Molecular type: Peptide
Sequence:
Gly Trp Gly Leu Gly Lys
1               5
SEQ ID NO: 2
Sequence type: Amino acid
Sequence length: 17
Topology: Linear
Molecular type: Peptide
Sequence:
Leu Leu Gln Asn Gln Ile Gln Gln Gln Thr Arg Thr Asp
1               5                   10
Glu Gly Thr Pro
    15
SEQ ID NO: 3
Sequence type: Nucleic acid
Sequence length: 11
Topology: Linear
Molecular type: Peptide
Sequence:

Thr Asp Lys Gly Trp Ser Phe Leu Leu Gly Lys
1               5                   10
SEQ ID NO: 4
Sequence type: Amino acid
Sequence length: 13
Topology: Linear
Molecular type: Peptide
Sequence:
Phe Phe Leu Asn Met Lys Pro Glu Ile Gln Pro Gln Asp
1               5                   10

## Claims

1. An isolated leucine aminopeptidase having the following properties:
A) Action and substrate specificity:
(1) acts to degrade a synthetic substrate L-leucyl-P-nitroanilide, and this activity being not inhibited by L-methionine;
(2) acts to degrade at least oxytocin, vasopressin and angiotensin III among naturally occurring hormones;
B) Optimum pH and stable pH range: having the optimum pH in a neutral range (pH about 7.4);
C) Condition for inactivation by pH and temperature: in activated at 70°C (30 minutes);
D) Inhibition, activation and stabilization: inactivated by divalent metal ions such as Zn²⁺, Co²⁺, Ni²⁺, or the like;
E) Molecular weight: about 490 kDa (by gel filtration method); subunit, about 210 kDa (by SDS-PAGE method) (there is no difference in mobility between reducing conditions and non-reducing conditions); and
F) containing the amino acid sequences shown in SEQ ID NOs: 1, 2, 3 and 4.
